# EUROPEAN PATENT APPLICATION

(11) **EP 2 567 705 A2**
(43) Date of publication of application: **13.03.2013**
(21) Application number: 12177882.3
(22) Date of filing: 04.06.2003
(51) Int. Cl.: A61K 38/00, A61K 31/195, A61K 31/155, A61K 47/48, A61K 31/122, A61K 31/198, A61K 31/205, A61K 31/4164, A61K 31/7004, A61K 31/7076, A61K 31/14, A61K 31/191, A61K 31/197

(54) **Methods of treating cognitive dysfunction by modulating brain energy metabolism**

(30) Priority: 04.06.2002 US 385836 P
(62) Divisional of application: 03734387.8
(71) Applicant: Avicena Group, Inc., South San Francisco, CA 94080 (US)
(72) Inventor: Kaddurah-Daouk, Rima, Belmont, Masachusetts 02178 (US); Clark, Joseph, F., South San Francisco, CA 94080 (US); Degrauw, Ton, South San Francisco, CA 94080 (US)
(74) Representative: Nash, David Allan

(57) **Abstract**

The invention relates to a brain energy modulating compound for use in:
(a) a method for treating a cognitive dysfunction in a subject, which method comprises administering an effective amount of the brain energy modulating compound such that said cognitive dysfunction is treated; or
(b) a method for treating a cognitive dysfunction in a subject by modulating the subject's brain pH such that the cognitive dysfunction is treated; or
(c) a method for treating creatine transport dysfunction in a subject, which method comprises administering an effective amount of said brain energy modulating compound such that said cognitive dysfunction is treated.

## Description

### Related Application

This application claims priority to U.S. Provisional Patent Application Serial No. 60/385,836, filed on June 4, 2002, the entire contents of which are hereby incorporated herein by reference in its entirety.

### Background of the Invention

Creatine is synthesized mainly in liver and kidney. L-arginine:glycine amidinotransferase (AGAT; EC 2.1.4.1) is involved in the formation of guanidino-acetate (GAA) from arginine and glycine. GAA is methylated by S-adenosyl-L-methionine:N-guanidinoacetate methyltransferase (GAMT; EC 2.1.1.2) to form creatine. While some creatine can come from the diet, about 1-2 grams of creatine is synthesized in liver and kidney per day. Creatine, as a dietary component, is found in many red meats and is readily absorbed from the gut. It is transported through the bloodstream to the target tissues, where it is taken up, against a large concentration gradient, by a saturable, Na⁺ dependent creatine transporter that spans the plasma-membrane. Inside the cell, creatine takes part in the energy metabolism through the creatine kinase reaction and it is metabolized at a constant rate to creatinine, which is excreted through the kidneys. About 3% of the total body creatine is lost per day in this way. This 3% is independent of the amount of creatine in the body, so if there is creatine supplementation that increases total body creatine, the creatinine excretion is predicted to be increased as well.

Studies on creatine transport have focused on the influx of creatine in several different tissues (Ku, C.-P. Biochim. Biophys. Acta. 600:212-227, 1980; Loike, J. D., Am.J.Physio/. 251:C128-C135, 1986; Möller, A. J.Neurochem 52:544-550, 1989) (See Figure 3-6). Transport is highly specific, Na⁺ dependent, and sensitive to metabolic inhibitors (Fitch, C. D. et al. Neurology 18:32-42, 1968; Fitch, C. D. Metabolism 29:686-690, 1980; Loike, J. D. et al. Clinical Research 34:548, 1986; Loike, J. D. et al. Proc. Natl. Acad. Sci. USA. 85:807-811, 1988; Möller, A. J.Neurochem 52: 544-550, 1989). In the rat blood stream, the concentration of creatine is about 100 µM (Syllm-Rapoport, I. et al. Acta Biol. Med. Germ. 40:653-659, 1980) while the intracellular concentration is several milimolar. Data from human monocytes and macrophages shows the Kₘ in the normal cells to be approximately 30 µM. The creatine concentration in human serum is in the range of 50 µM. Thus, the transporter in these human cells can respond to physiological fluctuations in creatine by altering the activity of the transporter.

### Summary of the Invention

In an embodiment, the invention pertains, at least in part, to a method for treating a cognitive dysfunction in a subject, by administering to the subject an effective amount of a brain energy modulating compound, such that the cognitive dysfunction in the subject is treated.

In another embodiment, the invention pertains, at least in part, to a method for the treatment of cognitive dysfunction in a subject. The method includes administering to the subject an effective amount of a creatine compound-protein conjugate to treat the cognitive dysfunction in the subject.

The invention also pertains, at least in part, to pharmaceutical compositions, comprising an effective amount of a creatine compound-protein conjugate and a pharmaceutically acceptable carrier. The invention also pertains to creatine compound-protein conjugates as a composition of matter.

In another embodiment, the invention pertains, at least in part, to a method for treating cognitive dysfunction in a subject. The method includes administering to a subject an effective amount of a creatine compound or creatine analogue, such that the cognitive dysfunction is treated.

In yet another embodiment, the invention pertains, at least in part, to a method for the treating cognitive dysfunction in a subject. The method includes modulating the subject's brain pH.

### Brief Description of the Drawings

Figure 1A is a digital image of a MRI of a subject's brain. The subject was subsequently diagnosed with a creatine transporter dysfunction.
Figure 1B is a Long Echo ¹H MR Spectrum of the subject's brain. The inset box of the MRI (Figure 1A) shows the voxel where the spectrum was obtained. The white matter shows a profound lack of creatine resonance.
Figure 2 is a schematic representation of mutations that have been observed in in *SLC6A8*/*CRTR1,* the creatine transporter protein.

### Detailed Description of the Invention

### Methods for Treating Cognitive dysfunction By Modulating Brain Energy Metabolism

Energy metabolism impairment is believed to be a component in cognitive dysfunction, behavioral and expressive deficiencies (Cecil, K. M. et al. Ann Neurol 49:401-4, 2001; Salomons, G. S. et al. Am J Hum Genet 68: 1497-500, 2001). The brain is dependent upon glucose oxidation for energy metabolism, and, to a lesser extent, it is also able to use ketone bodies as an energy source under certain conditions. The brain tightly controls energy metabolism and glucose oxidation to maintain an adequate energy supply.

In an embodiment, the invention pertains, at least in part, to a method for treating a cognitive dysfunction in a subject by modulating, e.g., increasing, brain energy metabolism. Brain energy metabolism can be modulated by administering to the subject an effective amount of a brain energy metabolism modulating compound. In a further embodiment, the subject's brain energy metabolism is normal, after the administration of the brain energy modulating compound.

The term "brain energy metabolism" includes aerobic metabolism, anaerobic metabolism, glycolytic metabolism, mitochondrial metabolism, and the generation of energy buffers such as adenylate kinase and creatine kinase, which generate energy in the brain. It also includes energy metabolism in the subject's neural or glial cells. Brain energy metabolism can be increased by increasing the ATP or creatine phosphate concentration, or by decreasing the concentration of ADP, GDP, AMP, or other mono- or di-phosphorylated nucleotides. Brain metabolism can be increased by the administration of brain energy modulating compounds.

The term "cognitive dysfunction" includes learning dysfunction, autism, attention deficit disorders, fragile X syndrome, obsessive-compulsive disorders, speech dysfunction, speech deficits, learning disabilities, impaired communication skills, mental retardation, low IQ, and inborn errors of metabolism affecting the brain (such as, but not limited to creatine transporter dysfunction, GAMT, and AGAT). Cognitive dysfunction also includes states of altered cognitive, expressive and behavioral function. In an embodiment, GAMT deficiency is not a cognitive dysfunction of the invention. In one embodiment, the term "cognitive dysfunction" does not include neurodegenerative disorders.

The term "subject" includes cells and animals capable of suffering from cognitive dysfunction. It includes organisms which are at risk of suffering from cognitive dysfunction or who are currently suffering from cognitive dysfunction. Examples of organisms include both transgenic and non-transgenic rodents, goats, pigs, sheep, cows, horses, squirrels, bears, rabbits, monkeys, chimpanzees, gorillas, frogs, fish, birds, cats, dogs, ferrets, and, preferrably, humans.

The term "creatine transporter dysfunction" includes a disorder charachterized by an inborn error creatine synthesis or of the creatine transporter or other abberant creatine transport function in the brain. The abberant creatine transport function in the brain may cause the subject to suffer from a low concentration of creatine in the brain of a subject suffering from creatine transporter dysfunction. In this disorder, impaired energy metabolism is believed to be associated with impaired learning dysfunction and cognitive function. It was found that treatments of similar neurological or cognitive dysfunctions do not tend to target improving metabolism and/or energy metabolism of the brain, neural cells, or glial cells. The invention also pertains, at least in part, to methods of treating subjects with a creatine transport deficiency in the brain.

The term "treating" includes the alleviation or diminishment of one or more symptoms of the disorder, disease, or dysfunction being treated. For example, for cognitive dysfunction may be treated by improving cognitive function, improving expressive function, decreasing seizure activity, improving behavioral parameters, increasing intelligence, or improving motor function.

The term "brain energy modulating compound" includes compounds which modulate the production or utlization of energy in the brain. Examples of brain energy modulating compounds include creatine compounds, creatine analogues, and other creatine compositions. Examples of creatine compounds include creatine phosphate, cyclocreatine (cCr), β-guanidinopropionic acid (βGPA), the acid anhydride of creatine-pyruvate (Cr-Py), the acid anhydride of creatine-glutamine (Cr-Gl), creatine glutamine, creatine-pyruvate, the acid anhydride of β-hydroxybutyrate (Cr-HB), creatine acetate, creatine phosphate, creatine beta-hydroxybutyrate, creatine choline, creatine compound-protein conjugates, and the ester of creatine-adenosine (Cr-Ado). Other brain energy modulating compounds include adenosine, acetoacetate, betahydroxybutyrate, gluconate, glycerate, fructose, fructose 1 phosphate, fructose 1-6 bisphostate, uridine diphosphosphoglucose, glucose 1 phosphate, glucose 6 phosphate, 3 phosphoglycerate, and 1-3 bisphosphoglycerate, phosphocreatine carnitine, arginine, pyruvate, and glutamine. Other brain energy modulating compounds include creatine choline, creatine β-hydroxybutyrate, creatine carnitine, creatine propionyl-carnitine, creatine Coenzyme Q10, creatine adenosine, creatine citrate, creatine pyruvate, creatine fructose, creatine fructose 1-6 bisphosphate, creatine gluconate,creatine, choline, β-hydroxybutyrate, carnitine, propionyl-carnitine, Coenzyme Q10, adenosine, citrate pyruvate, fructose, fructose 1-6 bisphosphate, and gluconate. The brain energy modulating compounds may be individual salts, complexes, or conjugates, and may be administered alone or in combination with one or more brain energy modulating compounds. Compounds which may be administered in combination with the brain energy modulating compounds include adenosine, pyruvate, and ketones.

The term "creatine analogue" includes compounds of the formula: 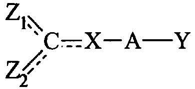 and pharmaceutically acceptable salts thereof, wherein:
a) Y is selected from the group consisting of: -CO₂H, -NHOH, -N0₂, - SO₃H, -C(=0)NHS0₂J and -P(=O)(OH)(OJ), wherein J is selected from the group consisting of: hydrogen, C₁-C₆ straight chain alkyl, C₃-C₆ branched alkyl, C₂-C₆ alkenyl, C₃-C₆ branched alkenyl, and aryl;
b) A is selected from the group consisting of: C, CH, C₁-C₅alkyl, C₂-C₅alkenyl, C₂-C₅alkynyl, and C₁-C₅ alkoyl chain, each having 0-2 substituents which are selected independently from the group consisting of:
   1) K, where K is selected from the group consisting of: C₁ -C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
   2) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy; and
   3) -NH-M, wherein M is selected from the group consisting of: hydrogen, C₁-C₄ alkyl, C₂-C₄ alkenyl, C₁-C₄ alkoyl, C₃-C₄ branched alkyl, C₃-C₄ branched alkenyl, and C₄ branched alkoyl;
c) X is selected from the group consisting of NR₁, CHR₁, CR₁, O and S, wherein R₁ is selected from the group consisting of:
   1) hydrogen;
   2) K where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
   3) an aryl group selected from the group consisting of a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
   4) a C₅-C₉ a-amino-w-methyl-w-adenosylcarboxylic acid attached via the w-methyl carbon;
   5) a C₅-C₉ a-amino-w-aza-w-methyl-w-adenosylcarboxylic acid attached via the w-methyl carbon; and
   6) a C₅-C₉ a-amino-w-thia-w-methyl-w-adenosylcarboxylic acid attached via the w-methyl carbon;
d) Z₁ and Z₂ are chosen independently from the group consisting of: =0, -NHR₂, -CH₂R₂, -NR₂OH; wherein Z₁ and Z₂ may not both be =0 and wherein R₂ is selected from the group consisting of:
   1) hydrogen;
   2) K, where K is selected from the group consisting of: C₁-C₆ straight alkyl; C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
   3) an aryl group selected from the group consisting of a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
   4) a C₄-C₈ a-amino-carboxylic acid attached via the w-carbon;
   5) B, wherein B is selected from the group consisting of: -CO₂H, NHOH, -SO₃H, -N0₂, OP(=O)(OH)(OJ) and -P(=O)(OH)(OJ), wherein J is selected from the group consisting of: hydrogen, C₁-C₆ straight alkyl, C₃-C₆ branched alkyl, C₂-C₆ alkenyl, C₃-C₆ branched alkenyl, and aryl, wherein B is optionally connected to the nitrogen via a linker selected from the group consisting of: C₁-C₂ alkyl, C₂ alkenyl, and C₁-C₂ alkoyl;
   6) -D-E, wherein D is selected from the group consisting of: C₁-C₃ straight alkyl, C₃ branched alkyl, C₂-C₃ straight alkenyl, C₃ branched alkenyl, C₁-C₃ straight alkoyl, aryl and aroyl; and E is selected from the group consisting of: -(P0₃)ₙNMP, where n is 0-2 and NMP is ribonucleotide monophosphate connected via the 5'-phosphate, 3'-phosphate or the aromatic ring of the base; -[P(=O)(OCH₃)(0)]ₘ-Q, where m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose or the aromatic ring of the base; and an aryl group containing 0-3 substituents chosen independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁ -C₆ straight alkoyl, C₃-C₆ branched alkyl, C₃-C₆ branched alkenyl, C₄-C₆ branched alkoyl, wherein E may be attached to any point to D, and if D is alkyl or alkenyl, D may be connected at either or both ends by an amide linkage; and
   7) -E, wherein E is selected from the group consisting of - (P0₃)ₙNMP, where n is 0-2 and NMP is a ribonucleotide monophosphate connected via the 5'-phosphate, 3'-phosphate or the aromatic ring of the base; -[P(=O)(OCH₃)(0)]ₘ-Q, where m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose or the aromatic ring of the base; and an aryl group containing 0-3 substituents chose independently from the group consisting of: C₁, Br, epoxy, acetoxy, -OG, -C(=O)G, and -CO=G, where G is independently selected from the group consisting of: C₁-C₆ straight alkyl, C₂-C₆ straight alkenyl, C₁-C₆ straight alkoyl, C₃-C₆ branched alkyl, C₃-C₆ branched alkenyl, C₄-C₆ branched alkoyl; and if E is aryl, E may be connected by an amide linkage;
e) if R₁ and at least one R₂ group are present, R₁ may be connected by a single or double bond to an R₂ group to form a cycle of 5 to 7 members;
f) if two R₂ groups are present, they may be connected by a single or a double bond to form a cycle of 4 to 7 members; and
g) if R₁ is present and Z₁ or Z₂ is selected from the group consisting of - NHR₂, -CH₂R₂ and -NR₂OH, then R₁ may be connected by a single or double bond to the carbon or nitrogen of either Z₁ or Z₂ to form a cycle of 4 to 7 members.

The term "creatine compound-protein conjugate" includes creatine compound- protein conjugates as well as creatine analogue-protein conjugates. The creatine compound-protein conjugates may comprise one or more creatine compounds or creatine analogues linked, e.g., covalently to a polypeptide . In a further embodiment, the creatine compounds and/or creatine analogues are linked to the protein or polypeptide through phosphoester linkages. A single creatine compound-protein conjugate may comprise one or more creatine compounds and/or creatine analogues, which may be the same or different.

In a further embodiment, the protein of the creatine compound-protein conjugate is a protein that is able to be transported into the brain, or into neural or glial cells. For example, the protein of the creatine compound-protein conjugate may be a sequence of about 11 amino acids (tyr-ala-arg-ala-ala-ala-arg-gln-ala-arg-ala). This sequence is known to be transported into the brain (neural and glial cells). Creatine compounds and creatine analogues can be attached to this protein through, for example, the N terminal, C terminal, hydroxy groups, and other reactive functional groups. The creatine compounds and analogues can be attached through reactive functional groups such as through carboxy and guanidino functional groups.

YARAAARQARA is a protein sequence which is capable of crossing the blood brain barrier. In one embodiment, the creatine compound is linked to the C terminal alanine of the protein. In a further embodiment, the conjugation of the creatine compound or analogue to the protein does substantially alter the secondary, tertiary or quaternary structure of the protein.

Examples of methods for conjugating creatine to the protein described above (YARAAARQARA) includes linking the carboxy group of the creatine to carboxy terminal of the protein; guanidino group of creatine to carboxy terminal of the protein; carboxy group of the creatine to the amino terminal of the protein; guanidino group of the creatine to the amino terminal of the protein; carboxy group of the creatine to the glutamine residue of the protein; and the guanidino group of the creatine to glutamine residue of the protein. There are numerous other structural combinations possible with conjugation at any of the reactive functional groups.

In a further embodiment, the creatine compound or creatine analogue comprises a phosphate group, such as creatine phosphate. The phosphate group of the creatine phosphate (or other phosphate containing creatine analogue or compound) could be used to link the creatine compound to the peptide. The phospho-ester linkage is a stable covalent bond that is readily hydrolyzed in the cell via esterases.

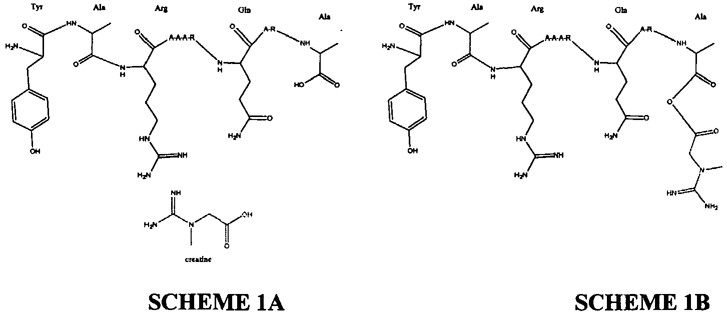

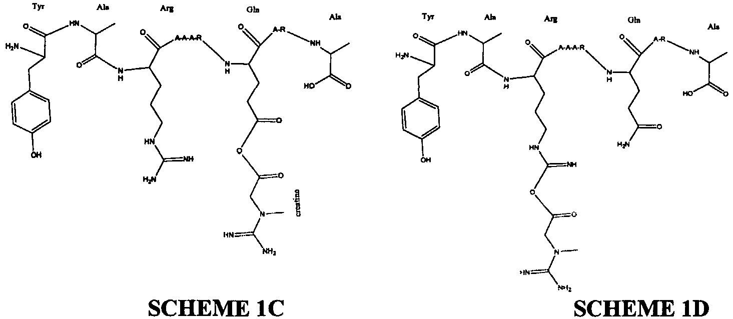

Scheme 1A-1D depicts an abbreviated schematic of an 11 amino acid peptide that has been found to cross the blood brain barrier. Scheme 1A depicts a peptide with the structure of creatine immediately below it. The amino acid residues Tyr, Ala, Arg, Gln and the C-terminal Ala may function as putative binding sites for the creatine. In Schemes 1B-1D, the attachment of creatine through readily hydrolyzable acid anhydride bonds is shown. The structure in Scheme 1B shows creatine being attached to the carboxy terminus of the peptide via an acid anhydride bond with the creatine.

Preferrably, the creatine compound-protein conjugate will allow for the creatine compound or analogue to get into brain cells. In one embodiment, after being transported into the brain, the protein may be degraded, *e.g*., by peptidases or exopeptidases, and the creatine compound or creatine analogue would be located within the brain to modulate brain energy metabolism. In a further embodiment, the protein is a Rijong-Ran polypeptide.

The invention pertains at least in part to the creatine compound-protein conjugates described herein as well as methods of using the creatine compound-protein conjugates to treat cognitive dysfunction, modulate brain energy metabolism, or modulate brain pH.

In one embodiment, the protein of the creatine compound-protein conjugate comprises 2 or more, 3 or more, 4 or more, 5 or more, 6 or more, 7 or more, 8 or more, 9 or more, 10 or more amino acid residues. In another embodiment, the protein of the creatine compound-protein conjugate comprises 100 or less, 80 or less, 70 or less, 60 or less, or 50 or less amino acid residues. In another embodiment, the creatine compound of the creatine compound-protein conjugate is a creatine analogue. In another embodiment it is creatine, creatine phosphate, cyclocreatine or another brain energy modulating compound described herein.

### Pharmaceutical Compositions for the Treatment of Cognitive dysfunctions

The brain energy modulating compounds may be administered to the subject in combination with a pharmaceutically acceptable carrier.

The phrase "pharmaceutically acceptable carrier" includes a pharmaceutically acceptable material, composition or vehicle, such as a liquid or solid filler, diluent, excipient, solvent or encapsulating material, involved in carrying or transporting a compound(s) of the invention within or to the subject such that it can performs its intended function. Typically, such compounds are carried or transported from one organ, or portion of the body, to another organ, or portion of the body. Each carrier must be "acceptable" in the sense of being compatible with the other ingredients of the formulation and not injurious to the subject. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

As set out above, certain embodiments of the present compounds can contain a basic functional group, such as amino or alkylamino, and are, thus, capable of forming pharmaceutically acceptable salts with pharmaceutically acceptable acids. The term "pharmaceutically acceptable salts" in this respect, refers to the relatively non-toxic, inorganic and organic acid addition salts of compounds of the invention. These salts can be prepared *in situ* during the final isolation and purification of the compounds of the invention, or by separately reacting a purified compound of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, *e.g*., Berge et al. (1977) "Pharmaceutical Salts", J. Pharm. Sci. 66:1-19).

In other cases, the compounds of the invention may contain one or more acidic functional groups and, thus, are capable of forming pharmaceutically acceptable salts with pharmaceutically acceptable bases. The term "pharmaceutically acceptable salts" in these instances refers to the relatively non-toxic, inorganic and organic base addition salts of compounds of the invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

Formulations of the invention include those suitable for oral, nasal, topical, transdermal, buccal, sublingual, rectal, vaginal and/or parenteral administration. The formulations may conveniently be presented in unit dosage form and may be prepared by any methods well known in the art of pharmacy. The amount of active ingredient which can be combined with a carrier material to produce a single dosage form will generally be that amount of the compound which produces a therapeutic effect.

Methods of preparing these formulations or compositions include the step of bringing into association a compound of the invention with the carrier and, optionally, one or more accessory ingredients. In general, the formulations are prepared by uniformly and intimately bringing into association a compound of the invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the invention as an active ingredient. A compound of the invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alginates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert dilutents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. Besides inert dilutents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the active compound in a polymer matrix or gel.

Pharmaceutical compositions of this invention suitable for parenteral administration comprise one or more compounds of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a compound, it is desirable to slow the absorption of the compound from subcutaneous or intramuscular injection. This may be accomplished by the use of a liquid suspension of crystalline or amorphous material having poor water solubility. The rate of absorption of the compound then depends upon its rate of dissolution which, in turn, may depend upon crystal size and crystalline form. Alternatively, delayed absorption of a parenterally-administered compound form is accomplished by dissolving or suspending the compound in an oil vehicle.

Injectable depot forms are made by forming microencapsule matrices of the compounds of the invention in biodegradable polymers such as polylactide-polyglycolide. Depending on the ratio of compound to polymer, and the nature of the particular polymer employed, the rate of compound release can be controlled. Examples of other biodegradable polymers include poly(orthoesters) and poly(anhydrides). Depot injectable formulations are also prepared by entrapping the drug in liposomes or microemulsions which are compatible with body tissue.

The preparations of the invention may be given orally, parenterally, topically, or rectally. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systematically," "peripheral administration" and "administered peripherally" as used herein mean the administration of a compound, drug or other material other than directly into the central nervous system, such that it enters the subject's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally and topically, as by powders, ointments or drops, including buccally and sublingually.

Regardless of the route of administration selected, the compounds of the invention, which may be used in a suitable hydrated form, and/or the pharmaceutical compositions of the invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular subject, composition, and mode of administration, without being toxic to the subject.

The selected dosage level will depend upon a variety of factors including the activity of the particular compound of the invention employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the subject being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

The regimen of administration can affect what constitutes an effective amount. The brain energy modulating compound can be administered to the subject either prior to or after the onset of a cognitive dysfunction. Further, several divided dosages, as well as staggered dosages, can be administered daily or sequentially, or the dose can be continuously infused, or can be a bolus injection. Further, the dosages of the brain energy modulating compounds can be proportionally increased or decreased as indicated by the exigencies of the therapeutic or prophylactic situation.

Creatine use is widespread among athletes, including adolescents with dosages up to 30 gms/day. The therapeutic dose in the creatine deficient subjects is not known and may depend upon the specific diagnosis and/or metabolic defect. Nonetheless, the creatine transporter dysfunction carriers are also candidates for benefiting from creatine supplementation to optimize intracellular creatine levels. It is not known if creatine transporter deficient subjects can take up creatine at higher blood concentrations. For these subjects very high levels of creatine supplementation or alternate metabolic therapies are required (or gene therapy).

In one embodiment, the invention pertains to compositions comprising brain energy modulating compounds. The compositions may comprise an effective amount of one or more brain energy modulating compounds to treat cognitive dysfunction in a subject. The compositions further may comprise a pharmaceutically acceptable carrier.

The invention also pertains to methods of treating cognitive dysfunction in a subject, by administering to the subject an effective amount of a combination of a creatine compound and a supplemental compound, such that the cognitive dysfunction is treated.

The term "effective amount" includes the amount of a brain energy modulating compound necessary for the treatment, amelioration, or prevention of at least one symptom of cognitive dysfunction.

### Methods of Treating Cognitive dysfunction by Increasing Creatine Production in the Brain

There are reports that rodent brains can synthesize creatine (Braissant, O. et al. Brain Res Mol Brain Res 86:193-201, 2001; Dringen, R. et al. J Neurochem 70:835-40, 1998). These studies suggested that rat brain cells had the enzymes that could synthesize creatine. However, it was not clear if there was sufficient synthetic activity in the rat brains to make and maintain normal levels of creatine. Moreover, the culture techniques used in these studies could mean that the cells were immature or de-differentiated. Neonatal rat brain cells may not have finished developing the creatine transport system and therefore depend on nascent synthetic activities for creatine. The adult rodent brain might, therefore, stop synthesizing creatine as part of the developmental process and eventually depend upon creatine transport for the brain's creatine supply.

If it is found that mammalian brains can synthesize creatine, the enzymes that make creatine could become therapeutic targets to treat cognitive dysfunction. Furthermore, cognitive dysfunctional also may be treated by increasing GAMT activity, by administering an agent which modulates its activity. The agent which modulates GAMT activity could be an agonist, or protein transfection based on the RijongRan peptide or other peptides readily transported into the brain. GAMT activity could also be modulated through gene engineering.

In another embodiment, the invention pertains to a method for treating cognitive dysfunction in a subject, by increasing the concentration of creatine compounds, such as creatine or creatine phosphate, in the subject's brain.

In another embodiment, the invention pertains to a method for treating cognitive dysfunction in a subject, by increasing the concentration of ATP in the subject's brain.

### Methods for Treating Cognitive dysfunction By Modulating Brain pH

The creatine kinase reaction is believed to be pH sensitive; it is believed that as the concentration of H⁺ ions increases, creatine phosphate hydrolysis is also increased. The creatine kinase and creatine phosphate system is sometimes considered to be a pH buffer in cells. For example, in periods of ischemia and acidosis, the acidosis is sometimes tempered (buffered) by the consumption of H⁺ ions during the hydrolysis of creatine phosphate. Brain acidosis has been associated with lower IQ (Rae, C. et al. Neurology 51: 33-40, 1998; Rae, C. et al. Proc R Soc Lond B Biol Sci 263: 1061-4, 1996; Tracey, I. et al. Lancet 345:1260-4, 1995).

Not to be limited by theory, the lack of brain creatine/creatine phosphate may impede the brain's ability to buffer pH changes and thereby result in acidosis. Although brain pH has not yet been correlated to IQ in this subject population, brain metabolism (pH and creatine) has been associated with clinical conditions where cognitive function is impaired (Cecil, K. M. et al. Ann Neurol 49: 401-4, 2001; Rae, C. et al. Neurology 51: 33-40., 1998; Rae, C. et al. Proc R Soc Lond B Biol Sci 263: 1061-4, 1996; Salomons, G. S. et al. Am JHum Genet 68: 1497-500,2001). Abnormal pH may cause abnormal brain metabolism, because many metabolic enzymes are pH sensitive, and abnormal metabolism may alter pH or pH buffering.

In one embodiment, the invention pertains to methods for treating cognitive dysfunction in a subject, by modulating the subject's brain pH, such that the cognitive dysfunction in the subject is treated. The subject's brain pH can be modulated by, for example, altering brain energy metabolism by the administration of a brain energy modulating compound, as described above.

### Methods of Diagnosing and Montoring Cognitive dysfunction

In another embodiment, the invention pertains to methods and kits for diagnosing cognitive dysfunction or abnormal brain energy metabolism by measuring blood, serum or plasma intracellular and extracellular metabolite concentrations.

In yet another embodiment, the invention pertains to methods and kits for the diagnosis of errors in creatine metabolism and creatine transport, by measuring the level of creatine or another brain metabolite in the blood serum, plasma, or urine. The diagnostic test may be used to diagnose the condition (or carrier status) and assess therapeutic treatments of subjects who have defects in creatine metabolism or transport and to follow the course of the disease.

In yet another embodiment, the invention pertains to a method for diagnosing errors in creatine metabolism and creatine transport by measuring creatine in the blood cell and in the serum/plasma. The diagnostic test may be used to diagnose the disease or carrier status of the disease and to assess the whole body status of creatine and metabolites, as well as the intracellular creatine and metabolites. The blood cells (Red blood cells, white blood cells etc) will reflect the transport activity and metabolic changes occurring in the brain and other tissues.

In yet another embodiment, the invention pertains to a method for diagnosis of cognitive dysfunction in a subject, by measuring the concentration of metabolites of creatine in a body sample. The body sample can be from the subject's blood stream, whole blood, blood cells, serum, plasma, tissue biopsy, cerebral spinal fluid, or other diagnostic samples. Examples of creatine metabolites include but are not limited to; creatine, creatinine, guanidine, guanidine acetic acid, arginine, methionine, homocistine, phosphocreatine and the relative ratios therein. Other metabolites and ratio comparisons will be known to those experienced in the art.

In another embodiment, the invention pertains to a method for diagnosing diseases of creatine transport. The method includes measuring the intracellular creatine in a body sample, (e.g., the subject's blood cells (RBC, WBC, etc.) or biopsy from the subject (fibroblast, skin, muscle, brain, etc.)). The method can be used to diagnose the condition (including carrier status), and assess therapeutic treatments of subjects who have defects in creatine metabolism or transport and to follow the course of the disease. Creatine levels can be detected using any method known in the art, such as, NMR, MRS, HPLC, antibodies, enzyme linked assays, and spectrophotometric assays.

In another embodiment, the invention pertains to method for diagnosing creatine transport dysfunction by measuring the level of the creatine transporter protein or protein fragments or derivatives thereof. The measurement of the creatine transporter protein can be accomplished by western blot, southern blot, oligos or ELISA. The tests can be done with blood cells, skin cells or other biopsy material known to those skilled in the art.

In another embodiment, the invention includes a method for diagnosing a cognitive dysfunction by using a blood or blood urine test to measure serum and cellular metabolites relevant to brain energy metabolism. Creatine has been measured in the serum and blood cells, and it appears to correlate with the changes seen in the subjects. The results from these studies suggest that the blood and urine tests are an index of transporter activity. Also the difference in circulating creatine in the serum and the concentration in the blood cells may be a quantifiable index of the activity of the creatine transporter. For example, total creatine concentration in the red blood cell when it is released from the bone marrow may be about 1 mm while serum free creatine is about 50 micro molar. If it is assumed that it will take about 30 days for the blood cells to lose their original creatine, and the average red blood cell 'lives' 100 days, then there will be decreased creatine in the blood cells, or a decreased blood cell:serum ratio. The red blood cell creatine is predicted to be decreased because of decreased creatine transport activity.

In another embodiment, the invention pertains to a method for determining a subject's tolerance to the administration of a creatine compound. The creatine tolerance test comprises comparing pre-oral creatine compound levels to post oral creatine compound levels. The method includes measuring the amount of creatine compound increase in the serum and in the blood cells. It is believed that subjects with a creatine transporter defect, or absence, would have impaired increases in the blood cell creatine compound concentration.

There are no commercial kits to diagnose creatine transporter dysfunction. All work to date has been accomplished by those experienced in the art to diagnose these subjects and carriers on a case-by-case basis with multiple modalities (MRS/MRI/Western-blot etc). There are publications where the creatine kinase has been knocked out. There are subjects with synthesis defects of creatine. However, the creatine kinase knockout is only in animals. Should there be subjects with this disease it would be very rare because it would require at least two simultaneous mutations to remove both creatine kinase gene products. The creatine synthesis defect subjects have improvement with creatine supplementation, but various cognitive dysfunction persists. The reasons for this are unclear, but it could be due to the increased levels of toxic guanidines that are seen in these subjects, or that the damage is done prior to creatine administration.

In a further embodiment, the invention pertains to a method for diagnosing cognitive dysfunction, by measuring brain and blood energy metabolism/metabolites. It is believed that by measuring brain and blood energy metabolism/metabolites the therapeutic efficacy of the therapeutic strategies can be assessed.

The strategy for the treatment of the subject may depend on the particular subject and the particular cognitive dysfunction. For example, a heterozygous female carrier of creatine transporter dysfunction may benefit from high doses of creatine administration to increase cognitive function. Increased cognitive function may be manifested as increased IQ or expressive improvements. Second, the males with creatine transporter dysfunction may benefit from high dose creatine administration, because the transporter protein may be present but with decreased activity. Subjects without the creatine transporter protein are not likely to benefit from the creatine therapy, and may require other therapeutic strategies such as administration of a creatine compound-protein conjugate.

### Models of Cognitive dysfunction

In an embodiment, the invention pertains to a method of modeling neurological disorders by impairing energy metabolism in the brain of an animal model or in cells. The animal or cellular model may be engineered to decrease phosphorylation potential, block substrate utilization, etc.

In another embodiment, the invention pertains, at least in part, to brain energy metabolism models of human cognitive dysfunction. The models may have altered brain creatine concentration or metabolism. The animal models may have deleted or modulated creatine transport or metabolism in their brains. Cells and cell cultures from these animals may also be used to model and correlate to the cognitive dysfunction. The animal models, cells, and cell lines can be used for testing therapies. In addition, cells from subjects suffering from neurological diseases can also be used to model and study cognitive dysfunction, e.g., to identify novel therapies. The cell lines generated from any one of these models may be immortalized.

### Exemplification of the Invention

The following example shows how the creatine transporter dysfunction can be diagnoses and treated using the methods of the invention.

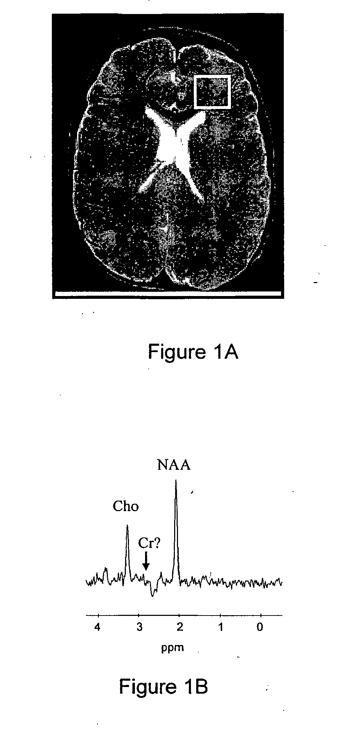

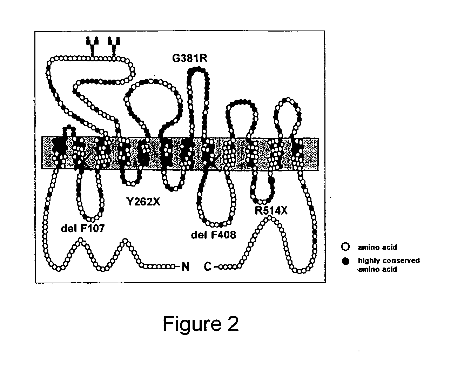

An 8 year old boy with creatine deficiency of the brain was diagnosed by proton MR Spectroscopy, as shown in Figures 1A and 1B. Upon further analysis, it was found that he has a nonsense mutation in the X-linked Creatine Transporter gene (CT1;SLC6A8) resulting in a shortened Cr Transporter protein as shown in Figure 2. Several female family members were identified as heterozygote carriers of this disorder, and appear to have decreased Cr. The boy had severe expressive dysphasia with other cognitive functions less affected.

The boy had severe expressive dysphasia with other cognitive functions less affected. The boy was treated with increasing doses of creatine to 750 mg/kg/day without clinical or spectroscopic (¹H MRS) improvement. In the absence of demonstrable benefit, the creatine treatment was discontinued after 6 months.

### Equivalents

Those skilled in the art will recognize, or be able to ascertain using no more than routine experimentation, many equivalents to the specific embodiments of the invention described herein. Such equivalents are intended to be encompassed by the following claims.

The entire contents of all references, patents, and patent applications cited herein are expressly incorporated by reference.

For the avoidance of doubt, the subject-matter of the present invention includes subject-matter as defined in the following numbered paragraphs (hereafter "para."):
1. A method for treating a cognitive dysfunction in a subject, comprising administering to said subject an effective amount of a brain energy modulating compound, such that said cognitive dysfunction in said subject is treated.
2. The method of para. 1, wherein said cognitive dysfunction is learning dysfunction, autism, attention deficit disorders, fragile X syndrome, obsessive-compulsive disorders, speech dysfunction, speech deficits, learning disabilities, impaired communication skills, mental retardation, low IQ, and inborn errors of metabolism affecting the brain.
3. The method of para. 1, wherein said subject is a human.
4. The method of para. 3, wherein said human is at risk of suffering from a cognitive dysfunction.
5. The method of para. 3, wherein said human is suffering from a cognitive dysfunction.
6. The method of para. 2, wherein said cognitive dysfunction is creatine transporter dysfunction.
7. The method of para. 1, wherein said brain energy modulating compound is a creatine compound or a creatine analogues.
8. The method of para. 7, wherein said creatine compound is creatine, creatine phosphate, cyclocreatine (cCr), β-guanidinopropionic acid (βGPA), the acid anhydride of creatine-pyruvate (Cr-Py), the acid anhydride of creatine-glutamine (Cr-Gl), creatine glutamine, creatine-pyruvate, the acid anhydride of β-hydroxybutyrate (Cr-HB), creatine acetate, creatine phosphate, creatine beta-hydroxybutyrate, creatine choline, creatine choline, creatine β-hydroxybutyrate, creatine carnitine, creatine propionyl-carnitine, creatine Coenzyme Q10, creatine adenosine, creatine citrate, creatine pyruvate, creatine fructose, creatine fructose 1-6 bisphosphate, creatine gluconate,or the ester of creatine-adenosine (Cr-Ado).
9. The method of para. 1, wherein said brain energy modulating compound is adenosine, acetoacetate, betahydroxybutyrate, gluconate, glycerate, fructose, fructose 1 phosphate, fructose 1-6 bisphostate, uridine diphosphosphoglucose, glucose 1 phosphate, glucose 6 phosphate, 3 phosphoglycerate, and 1-3 bisphosphoglycerate, phosphocreatine carnitine, arginine, pyruvate, glutamine, choline, β-hydroxybutyrate, carnitine, propionyl-carnitine, Coenzyme Q10, adenosine, citrate pyruvate, fructose, fructose 1-6 bisphosphate, or gluconate.
10. The method of para. 7, wherein said creatine analogue is a compound of the formula: 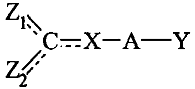 and pharmaceutically acceptable salts thereof, wherein:
   a) Y is selected from the group consisting of: -CO₂H, -NHOH, -N0₂, - SO₃H, -C(=O)NHS0₂J and -P(=O)(OH)(OJ), wherein J is selected from the group consisting of: hydrogen, C₁-C₆ straight chain alkyl, C₃-C₆ branched alkyl, C₂-C₆ alkenyl, C₃-C₆ branched alkenyl, and aryl;
   b) A is selected from the group consisting of: C, CH, C₁-C₅alkyl, C₂-C₅alkenyl, C₂-C₅alkynyl, and C₁-C₅ alkoyl chain, each having 0-2 substituents which are selected independently from the group consisting of:
      1) K, where K is selected from the group consisting of: C₁ -C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
      2) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy; and
      3) -NH-M, wherein M is selected from the group consisting of: hydrogen, C₁-C₄ alkyl, C₂-C₄ alkenyl, C₁-C₄ alkoyl, C₃-C₄ branched alkyl, C₃-C₄ branched alkenyl, and C₄ branched alkoyl;
   c) X is selected from the group consisting of NR₁, CHR₁, CR₁, O and S, wherein R₁ is selected from the group consisting of:
      1) hydrogen;
      2) K where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
      3) an aryl group selected from the group consisting of a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
      4) a C₅-C₉ a-amino-w-methyl-w-adenosylcarboxylic acid attached via the w-methyl carbon;
      5) a C₅-C₉ a-amino-w-aza-w-methyl-w-adenosylcarboxylic acid attached via the w-methyl carbon; and
      6) a C₅-C₉ a-amino-w-thia-w-methyl-w-adenosylcarboxylic acid attached via the w-methyl carbon;
   d) Z₁ and Z₂ are chosen independently from the group consisting of: =0, -NHR₂, -CH₂R₂, -NR₂OH; wherein Z₁ and Z₂ may not both be =0 and wherein R₂ is selected from the group consisting of:
      1) hydrogen;
      2) K, where K is selected from the group consisting of: C₁-C₆ straight alkyl; C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
      3) an aryl group selected from the group consisting of a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
      4) a C₄-C₈ a-amino-carboxylic acid attached via the w-carbon;
      5) B, wherein B is selected from the group consisting of: -CO₂H, - NHOH, -SO₃H, -N0₂, OP(=O)(OH)(OJ) and -P(=O)(OH)(OJ), wherein J is selected from the group consisting of: hydrogen, C₁-C₆ straight alkyl, C₃-C₆ branched alkyl, C₂-C₆ alkenyl, C₃-C₆ branched alkenyl, and aryl, wherein B is optionally connected to the nitrogen via a linker selected from the group consisting of: C₁-C₂ alkyl, C₂ alkenyl, and C₁-C₂ alkoyl;
      6) -D-E, wherein D is selected from the group consisting of: C₁-C₃ straight alkyl, C₃ branched alkyl, C₂-C₃ straight alkenyl, C₃ branched alkenyl, C₁-C₃ straight alkoyl, aryl and aroyl; and E is selected from the group consisting of: -(P0₃)ₙNMP, where n is 0-2 and NMP is ribonucleotide monophosphate connected via the 5'-phosphate, 3'-phosphate or the aromatic ring of the base; -[P(=O)(OCH₃)(0)]ₘ-Q, where m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose or the aromatic ring of the base; and an aryl group containing 0-3 substituents chosen independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁ -C₆ straight alkoyl, C₃-C₆ branched alkyl, C₃-C₆ branched alkenyl, C₄-C₆ branched alkoyl, wherein E may be attached to any point to D, and if D is alkyl or alkenyl, D may be connected at either or both ends by an amide linkage; and
      7) -E, wherein E is selected from the group consisting of - (P0₃)ₙNMP, where n is 0-2 and NMP is a ribonucleotide monophosphate connected via the 5'-phosphate, 3'-phosphate or the aromatic ring of the base; -[P(=O)(OCH₃)(0)]ₘ-Q, where m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose or the aromatic ring of the base; and an aryl group containing 0-3 substituents chose independently from the group consisting of: C₁, Br, epoxy, acetoxy, -OG, -C(=O)G, and -CO=G, where G is independently selected from the group consisting of: C₁-C₆ straight alkyl, C₂-C₆ straight alkenyl, C₁-C₆ straight alkoyl, C₃-C₆ branched alkyl, C₃-C₆ branched alkenyl, C₄-C₆ branched alkoyl; and if E is aryl, E may be connected by an amide linkage;
   e) if R₁ and at least one R₂ group are present, R₁ may be connected by a single or double bond to an R₂ group to form a cycle of 5 to 7 members;
   f) if two R₂ groups are present, they may be connected by a single or a double bond to form a cycle of 4 to 7 members; and
   g) if R₁ is present and Z₁ or Z₂ is selected from the group consisting of - NHR₂, -CH₂R₂ and -NR₂OH, then R₁ may be connected by a single or double bond to the carbon or nitrogen of either Z₁ or Z₂ to form a cycle of 4 to 7 members.
11. The method of para. 1, wherein said brain energy modulating compound is a creatine compound-protein conjugates.
12. The method of para. 11, wherein said creatine compound-protein conjugates comprises one or more creatine compounds or creatine analogues linked covalently to said protein.
13. The method of para. 12, wherein said covalent linkage is a phosphoester linkage.
14. The method of para. 11, wherein said creatine compound-protein conjugate is permeable through the blood brain barrier.
15. The method of para. 14, wherein said protein comprises the sequence tyr-ala-arg-ala-ala-ala-arg-gln-ala-arg-ala.
16. The method of para. 11, wherein said creatine compound-protein conjugate comprises one or more creatine compounds.
17. The method of para. 11, wherein said protein is broken down after crossing the blood brain barrier.
18. A method for the treatment of cognitive dysfunction in a subject, comprising administering to said subject an effective amount of a creatine compound-protein conjugate, such that said cognitive dysfunction in said subject is treated.
19. A pharmaceutical composition, comprising an effective amount of a creatine compound-protein conjugate and a pharmaceutically acceptable carrier.
20. The composition of para. 19, wherein said creatine compound is creatine or creatine phosphate.
21. The composition of para. 19, wherein said creatine compound is cyclocreatine (cCr), β-guanidinopropionic acid (βGPA), the acid anhydride of creatine-pyruvate (Cr-Py), the acid anhydride of creatine-glutamine (Cr-Gl), creatine glutamine, creatine-pyruvate, the acid anhydride of β-hydroxybutyrate (Cr-HB), creatine acetate, creatine phosphate, creatine beta-hydroxybutyrate, creatine choline, creatine choline, creatine β-hydroxybutyrate, creatine carnitine, creatine propionyl-carnitine, creatine Coenzyme Q10, creatine adenosine, creatine citrate, creatine pyruvate, creatine fructose, creatine fructose 1-6 bisphosphate, creatine gluconate,or the ester of creatine-adenosine (Cr-Ado).
22. The composition of para. 19, wherein said creatine compound-protein conjugate comprises a creatine analogue of the formula: 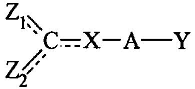 and pharmaceutically acceptable salts thereof, wherein:
   a) Y is selected from the group consisting of: -CO₂H, -NHOH, -N0₂, - SO₃H, -C(=O)NHS0₂J and -P(=O)(OH)(OJ), wherein J is selected from the group consisting of: hydrogen, C₁-C₆ straight chain alkyl, C₃-C₆ branched alkyl, C₂-C₆ alkenyl, C₃-C₆ branched alkenyl, and aryl;
   b) A is selected from the group consisting of: C, CH, C₁-C₅alkyl, C₂-C₅alkenyl, C₂-C₅alkynyl, and C₁-C₅ alkoyl chain, each having 0-2 substituents which are selected independently from the group consisting of:
      1) K, where K is selected from the group consisting of: C₁ -C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
      2) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy; and
      3) -NH-M, wherein M is selected from the group consisting of: hydrogen, C₁-C₄ alkyl, C₂-C₄ alkenyl, C₁-C₄ alkoyl, C₃-C₄ branched alkyl, C₃-C₄ branched alkenyl, and C₄ branched alkoyl;
   c) X is selected from the group consisting of NR₁, CHR₁, CR₁, O and S, wherein R₁ is selected from the group consisting of:
      1) hydrogen;
      2) K where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
      3) an aryl group selected from the group consisting of a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
      4) a C₅-C₉ a-amino-w-methyl-w-adenosylcarboxylic acid attached via the w-methyl carbon;
      5) a C₅-C₉ a-amino-w-aza-w-methyl-w-adenosylcarboxylic acid attached via the w-methyl carbon; and
      6) a C₅-C₉ a-amino-w-thia-w-methyl-w-adenosylcarboxylic acid attached via the w-methyl carbon;
   d) Z₁ and Z₂ are chosen independently from the group consisting of: =0, -NHR₂, -CH₂R₂, -NR₂OH; wherein Z₁ and Z₂ may not both be =0 and wherein R₂ is selected from the group consisting of:
      1) hydrogen;
      2) K, where K is selected from the group consisting of: C₁-C₆ straight alkyl; C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
      3) an aryl group selected from the group consisting of a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
      4) a C₄-C₈ a-amino-carboxylic acid attached via the w-carbon;
      5) B, wherein B is selected from the group consisting of: -CO₂H, - NHOH, -SO₃H, -N0₂, OP(=O)(OH)(OJ) and -P(=O)(OH)(OJ), wherein J is selected from the group consisting of: hydrogen, C₁-C₆ straight alkyl, C₃-C₆ branched alkyl, C₂-C₆ alkenyl, C₃-C₆ branched alkenyl, and aryl, wherein B is optionally connected to the nitrogen via a linker selected from the group consisting of: C₁-C₂ alkyl, C₂ alkenyl, and C₁-C₂ alkoyl;
      6) -D-E, wherein D is selected from the group consisting of: C₁-C₃ straight alkyl, C₃ branched alkyl, C₂-C₃ straight alkenyl, C₃ branched alkenyl, C₁-C₃ straight alkoyl, aryl and aroyl; and E is selected from the group consisting of: -(P0₃)ₙNMP, where n is 0-2 and NMP is ribonucleotide monophosphate connected via the 5'-phosphate, 3'-phosphate or the aromatic ring of the base; -[P(=O)(OCH₃)(0)]ₘ-Q, where m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose or the aromatic ring of the base; and an aryl group containing 0-3 substituents chosen independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁ -C₆ straight alkoyl, C₃-C₆ branched alkyl, C₃-C₆ branched alkenyl, C₄-C₆ branched alkoyl, wherein E may be attached to any point to D, and if D is alkyl or alkenyl, D may be connected at either or both ends by an amide linkage; and
      7) -E, wherein E is selected from the group consisting of - (P0₃)ₙNMP, where n is 0-2 and NMP is a ribonucleotide monophosphate connected via the 5'-phosphate, 3'-phosphate or the aromatic ring of the base; -[P(=O)(OCH₃)(0)]ₘ-Q, where m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose or the aromatic ring of the base; and an aryl group containing 0-3 substituents chose independently from the group consisting of: C₁, Br, epoxy, acetoxy, -OG, -C(=O)G, and -CO=G, where G is independently selected from the group consisting of: C₁-C₆ straight alkyl, C₂-C₆ straight alkenyl, C₁-C₆ straight alkoyl, C₃-C₆ branched alkyl, C₃-C₆ branched alkenyl, C₄-C₆ branched alkoyl; and if E is aryl, E may be connected by an amide linkage;
   e) if R₁ and at least one R₂ group are present, R₁ may be connected by a single or double bond to an R₂ group to form a cycle of 5 to 7 members;
   f) if two R₂ groups are present, they may be connected by a single or a double bond to form a cycle of 4 to 7 members; and
   g) if R₁ is present and Z₁ or Z₂ is selected from the group consisting of - NHR₂, -CH₂R₂ and -NR₂OH, then R₁ may be connected by a single or double bond to the carbon or nitrogen of either Z₁ or Z₂ to form a cycle of 4 to 7 members.
23. The pharmaceutical composition of para. 19, wherein said effective amount is effective to treat a cognitive dysfunction in a subject.
24. A method for treating cognitive dysfunction in a subject, comprising administering to said subject an effective amount of a creatine compound or creatine analogue, such that said cognitive dysfunction is treated.
25. The method of para. 24, wherein said cognitive dysfunction is learning dysfunction, cognitive dysfunction, autism, attention deficit disorders, fragile X syndrome, obsessive-compulsive disorders, speech dysfunction, speech deficits, learning disabilities, impaired communication skills, mental retardation, low IQ, and inborn errors of metabolism affecting the brain.
26. A method for the treating cognitive dysfunction in a subject, comprising modulating the subject's brain pH, such that the cognitive dysfunction in said subject is treated.
27. The method of para. 26, wherein said subject's brain pH is modulated by the administration of a brain energy modulating compound.
28. A method for treating creatine transporter dysfunction in a subject, comprising administering to said subject an effective amount of a brain energy modulating compound, such that said subject is treated.
29. The method of para. 28, wherein said brain energy modulating compound is a creatine compound-protein conjugate.

## Claims

1. A brain energy modulating compound for use in:
(a) a method for treating a cognitive dysfunction in a subject, which method comprises administering an effective amount of the brain energy modulating compound such that said cognitive dysfunction is treated; or
(b) a method for treating a cognitive dysfunction in a subject by modulating the subject's brain pH such that the cognitive dysfunction is treated; or
(c) a method for treating creatine transport dysfunction in a subject, which method comprises administering an effective amount of said brain energy modulating compound such that said cognitive dysfunction is treated.

2. The brain energy modulating compound for use as claimed in claim 1, wherein the compound is for use in a method for treating a cognitive dysfunction in a subject, which method comprises administering an effective amount of the brain energy modulating compound such that said cognitive dysfunction is treated.

3. The brain energy modulating compound for use as claimed in claim 2, wherein said cognitive dysfunction is learning dysfunction, autism, attention deficit disorders, fragile X syndrome, obsessive-compulsive disorders, speech dysfunction, speech deficits, learning disabilities, impaired communication skills, mental retardation, low IQ, and inborn errors of metabolism affecting the brain.

4. The brain energy modulating compound for use as claimed in claim 2, wherein said subject is a human.

5. The brain energy modulating compound for use as claimed in claim 4, wherein said human is at risk of suffering from a cognitive dysfunction.

6. The brain energy modulating compound for use as claimed in claim 4, wherein said human is suffering from a cognitive dysfunction.

7. The brain energy modulating compound for use as claimed in claim 1, wherein said cognitive dysfunction is creatine transporter dysfunction.

8. A brain energy modulating compound for use as claimed in any preceding claim, wherein the compound is cyclocreatine (cCr).

9. The brain energy modulating compound for use as claimed in any one of claims 1 to 7, wherein the brain energy modulating compound is adenosine, acetoacetate, betahydroxybutyrate, gluconate, glycerate, fructose, fructose 1 phosphate, fructose 1-6 bisphostate, uridine diphosphosphoglucose, glucose 1 phosphate, glucose 6 phosphate, 3 phosphoglycerate, and 1-3 bisphosphoglycerate, phosphocreatine carnitine, arginine, pyruvate, glutamine, choline, β-hydroxybutyrate, carnitine, propionyl-carnitine, Coenzyme Q10, adenosine, citrate pyruvate, fructose, fructose 1-6 bisphosphate, or gluconate.

10. The brain energy modulating compound for use as claimed in claims 1 to 7, wherein the brain energy modulating compound is a creatine compound or a creatine analogue.

11. The brain energy modulating compound for use as claimed in claim 10, wherein the brain energy modulating compound is a creatine compound selected from β-guanidinopropionic acid (βGPA), the acid anhydride of creatine-pyruvate (Cr-Py), the acid anhydride of creatine-glutamine (Cr-Gl), creatine glutamine, creatine-pyruvate, the acid anhydride of β-hydroxybutyrate (Cr-HB), creatine phosphate, creatine beta-hydroxybutyrate, creatine choline, creatine choline, creatine β-hydroxybutyrate, creatine carnitine, creatine propionyl-carnitine, creatine Coenzyme Q10, creatine adenosine, creatine pyruvate, creatine fructose, creatine fructose 1-6 bisphosphate, creatine gluconate,or the ester of creatine-adenosine (Cr-Ado).

12. The brain energy modulating compound for use as claimed in claim 10, wherein the brain energy modulating compound is a creatine analogue, wherein said creatine analogue is a compound of the formula: 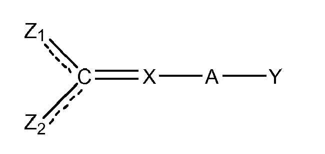 and pharmaceutically acceptable salts thereof, wherein:
a) Y is selected from the group consisting of: -CO₂H, -NHOH, -NO₂, -SO₃H, - C(=O)NHSO₂J and -P(=O)(OH)(OJ), wherein J is selected from the group consisting of: hydrogen, C₁-C₆ straight chain alkyl, C₃-C₆ branched alkyl, C₂-C₆ alkenyl, C₃-C₆ branched alkenyl, and aryl;
b) A is selected from the group consisting of: C, CH, C₁-C₅alkyl, C₂-C₅alkenyl, C₂-C₅alkynyl, and C₁-C₅alkoyl chain, each having 0-2 substituents which are selected independently from the group consisting of:
1) K, where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: rromo, chloro, epoxy and acetoxy;
2) an aryl group selected from the group consisting of: a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy; and
3) -NH-M, wherein M is selected from the group consisting of: hydrogen, C₁-C₄ alkyl, C₂-C₄ alkenyl, C₁-C₄ alkoyl, C₃-C₄ branched alkyl, C₃-C₄ branched alkenyl, and C₄ branched alkoyl;
c) X is selected from the group consisting of NR₁, CHR₁, CR₁, O and S, wherein R₁ is selected from the group consisting of:
1) hydrogen;
2) K where K is selected from the group consisting of: C₁-C₆ straight alkyl, C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
3) an aryl group selected from the group consisting of a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
4) a C₅-C₉ a-amino-w-methyl-w-adenosylcarboxylic acid attached via the w-methyl carbon;
5) a C₅-C₉ a-amino-w-aza-w-methyl-w-adenosylcarboxylic acid attached via the w-methyl carbon; and
6) a C₅-C₉ a-amino-w-thia-w-methyl-w-adenosylcarboxylic acid attached via the w-methyl carbon;
d) Z₁ and Z₂ are chosen independently from the group consisting of: =O, -NHR₂, - CH₂R₂, -NR₂OH; wherein Z₁ and Z₂ may not both be =O and wherein R₂ is selected from the group consisting of:
1) hydrogen;
2) K, where K is selected from the group consisting of: C₁-C₆ straight alkyl; C₂-C₆ straight alkenyl, C₁-C₆ straight alkoyl, C₃-C₆ branched alkyl, C₃-C₆ branched alkenyl, and C₄-C₆ branched alkoyl, K having 0-2 substituents independently selected from the group consisting of: bromo, chloro, epoxy and acetoxy;
3) an aryl group selected from the group consisting of a 1-2 ring carbocycle and a 1-2 ring heterocycle, wherein the aryl group contains 0-2 substituents independently selected from the group consisting of: -CH₂L and -COCH₂L where L is independently selected from the group consisting of: bromo, chloro, epoxy and acetoxy;
4) a C₄-C₈ a-amino-carboxylic acid attached via the w-carbon;
5) B, wherein B is selected from the group consisting of: -CO₂H-NHOH, - SO₃H, -NO₂, OP(=O)(OH)(OJ) and -P(=O)(OH)(OJ), wherein J is selected from the group consisting of: hydrogen, C₁-C₆ straight alkyl, C₃-C₆ branched alkyl, C₂-C₆ alkenyl, C₃-C₆ branched alkenyl, and aryl, wherein B is optionally connected to the nitrogen via a linker selected from the group consisting of: C₁-C₂ alkyl, C₂ alkenyl, and C₁-C₂ alkoyl;
6) -D-E, wherein D is selected from the group consisting of: C₁-C₃ straight alkyl, C₃ branched alkyl, C₂-C₃ straight alkenyl, C₃ branched alkenyl, C₁-C₃ straight alkoyl, aryl and aroyl; and E is selected from the group consisting of: -(PO₃)ₙNMP, where n is 0-2 and NMP is ribonucleotide monophosphate connected via the 5'-phosphate, 3'-phosphate or the aromatic ring of the base; - [P(=O)(OCH₃)(O)]ₘ-Q, where m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose or the aromatic ring of the base; and an aryl group containing 0-3 substituents chosen independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁-C₆ straight alkoyl, C₃-C₆ branched alkyl, C₃-C₆ branched alkenyl, C₄-C₆ branched alkoyl, wherein E may be attached to any point to D, and if D is alkyl or alkenyl, D may be connected at either or both ends by an amide linkage; and
7) -E, wherein E is selected from the group consisting of -(PO₃)ₙNMP, where n is 0-2 and NMP is a ribonucleotide monophosphate connected via the 5'-phosphate, 3'-phosphate or the aromatic ring of the base; -[P(=O)(OCH₃)(O)]ₘ-Q, where m is 0-3 and Q is a ribonucleoside connected via the ribose or the aromatic ring of the base; -[P(=O)(OH)(CH₂)]ₘ-Q, where m is 0-3 and Q is a ribonucleoside connected via the ribose or the aromatic ring of the base; and an aryl group containing 0-3 substituents chose independently from the group consisting of: Cl, Br, epoxy, acetoxy, -OG, -C(=O)G, and -CO₂G, where G is independently selected from the group consisting of: C₁-C₆ straight alkyl, C₂-C₆ straight alkenyl, C₁-C₆ straight alkoyl, C₃-C₆ branched alkyl, C₃-C₆ branched alkenyl, C₄-C₆ branched alkoyl; and if E is aryl, E may be connected by an amide linkage;
e) if R₁ and at least one R₂ group are present, R₁ may be connected by a single or double bond to an R₂ group to form a cycle of 5 to 7 members;
f) if two R₂ groups are present, they may be connected by a single or a double bond to form a cycle of 4 to 7 members; and
g) if R₁ is present and Z₁ or Z₂ is selected from the group consisting of -NHR₂, - CH₂R₂ and -NR₂OH, then R₁ may be connected by a single or double bond to the carbon or nitrogen of either Z₁ or Z₂ to form a cycle of 4 to 7 members.

13. A method for treating cognitive dysfunction in a subject, by increasing the concentration of ATP in the subject's brain.

14. A method which is selected from:
a method for the diagnosis of errors in creatine metabolism and creatine transport, by measuring the level of creatine or another brain metabolite in the blood serum, plasma, or urine;
a method for diagnosing errors in creatine metabolism and creatine transport by measuring creatine in the blood cell and in the serum/plasma;
a method for diagnosis of cognitive dysfunction in a subject, by measuring the concentration of metabolites of creatine in a body sample;
a method for diagnosing diseases of creatine transport including measuring the intracellular creatine in a body sample, (e. g. , the subject's blood cells (RBC, WBC, etc.) or biopsy from the subject (fibroblast, skin, muscle, brain, etc.));
a method for diagnosing creatine transport dysfunction by measuring the level of the creatine transporter protein or protein fragments or derivatives thereof;
a method for diagnosing a cognitive dysfunction by using a blood or blood urine test to measure serum and cellular metabolites relevant to brain energy metabolism.

15. A method of modeling neurological disorders by impairing energy metabolism in the brain of an animal model or in cells, wherein the animal or cellular model is engineered to decrease phosphorylation potential, block substrate utilization.
